# EUROPEAN PATENT APPLICATION

(11) **EP 2 520 279 A1**
(43) Date of publication of application: **07.11.2012**
(21) Application number: 11164400.1
(22) Date of filing: 02.05.2011
(51) Int. Cl.: A61K 9/00, A61K 9/06, A61K 47/10, A61K 47/36

(54) **Thermoreversible gel pharamaceutical compositions for odontoiatric use**

(71) Applicant: TERES S.r.l., 20121 Milan (IT); Mader S.r.l., 20123 Milano (IT)
(72) Inventor: Ceschel, Giancarlo, 20121 Milano (IT); Vandoni, Guido, 20151 Milano (IT)
(74) Representative: Gervasi, Gemma

(57) **Abstract**

The present invention concerns a topical pharmaceutical composition in form of thermoreversible gel comprising water, a topically active ingredient, a thickening matrix, based on one or more poloxamers, and hyaluronic acid. The composition of the invention, once applied to the gingival mucosa or to dental enamel, forms a gel having a slow release of the active ingredient incorporated therein.

## Description

### Field of the Invention

The present invention concerns a thermoreversible gel odontoiatric pharmaceutical composition.

The present invention is a pharmaceutical medical device for odontoiatric use.

### STATE OF THE ART

In odontology the use of pharmaceutical preparations containing locally active principles is very common. These preparations need prolonged contact times with the oral tissue in order to get the required therapeutical effect.

For example, during the treatment of gingival mucosa infections, antibacterial preparations are required to remain in contact with the infected mucosa until the local bacterial count decreases to acceptable levels.

A typical example is the bacterial infection of the root canals where the dental pulp is contained. As it is well known, the microorganisms proliferation will cause degeneration of the pulp and inflammation of the surrounding tissues.

In this case adequate cleaning of the root canals and local therapy with pharmaceutical preparations is needed.

Cleaning of the root canals and surrounding tissues can be obtained by mechanical devices able to remove the damaged tissues, followed by disinfection with specific washing solutions of the deepest areas of the root system, not reached by mechanical devices.

Disinfecting washing solutions, sometimes in a gel form, are generally used for endodontal irrigation and for the treatment of gingival mucosa diseases.

While root irrigation and pharmacological treatments of gingival mucosa are rather delicate operations, since the active substances can also play an aggressive action on the contacted tissues, at the same time it is crucial to keep the contact with gingival mucosa long enough to obtain the searched and desired effect.

In cases where the active substances stay in contact with the treated area for a limited period of time, as in the case of oral washing medical solutions, an appropriated therapeutical answer can be obtained by increasing the active principle concentration.

However the use of oral washing solutions at high active principle concentration may determine local side effects, such as mucosa irritation, reddening, itching or even superficial burns with allergic reactions in some cases.

Gel odontoiatric preparations are presently available as an alternative even if so far they have not yet come up to expectations.

Indeed the present gels for odontoiatric use, once in contact with the oral mucosa, tend to melt quickly at body temperature. The gel liquefies soon after application, is washed away from the site to be treated and cannot perform the expected pharmacological action.

Of course these phenomena are emphasized when the odontoiatric gel preparation is instable due to the active principle formulation.

At present and to our knowledge, in odontoiatric area the need of keeping the active principle in contact with the oral mucosa or with a tissue for a period of time sufficiently long to exert the appropriated therapeutic effect, is still unmet.

At the present time, there is need of an odontoiatric pharmaceutical formulation that keeps the active principle in contact with the mucosa or tissue to be treated long enough to achieve the required therapeutic effects.

One of the aims of the present invention is to provide odontoiatric pharmaceutical compositions that, once applied, remain active *in situ* for a required period of time in order to display the searched pharmacological therapeutic effect

Another aim of the present invention consists in providing odontoiatric pharmaceutical compositions having prolonged release times of the active principle, the use of which lowers the risk of local side effects.

### SUMMARY OF THE INVENTION

In accordance with one aspect of the present invention the Applicants found that a gel strongly sticks to the oral cavity mucose or to the dental enamel by adding a specific muco-adhesive agent to a topical thermoreversible gel composition, so extending the release time of the active principle.

According to a first aspect of the present invention a thermoreversible gel pharmaceutical formulation is hereafter described as containing:
i) water,
ii) a topical active principle,
iii) a thickening viscous matrix containing at least one poloxamer,
iv) a muco-adhesive agent that sticks the gel formulation to the oral mucosa or to the dental enamel
said formulation is characterized by the fact that the muco-adhesive agent is hyaluronic acid or its derivatives or its salts.

In the present invention, the term "gel" refers to an aqueous mass or colloidal system with a gelatinous aspect. More specifically, the term "thermoreversible gel" refers to systems obtained from aqueous (co)polymeric solutions, where the (co)polymer is a poloxamer, able to reversibly jelly in answer to changes of temperature. Typically, the jellying of the water polymeric solution can happen *in situ*, for instance as consequence of temperature increase when the co(polymeric) solution contacts a body tissue.

According to some preparations, the composition of the invention is liquid at temperatures inferior to room temperature and gelatinous at temperatures close to human body temperature. For instance, the composition of the invention is liquid at 5-15°C and gelatinous at temperatures around 37°C.

Within the present invention, the term poloxamer refers to block-copolymers (a copolymer formed with monomers of different length) essentially formed by three blocks: a central hydrophobic block of polypropylene oxide (PPO) joined at each end with a hydrophilic block of polyethylene oxide (PEO).

Typically poloxamers are obtained from sequential polymerization of propylene oxide (PO) and ethylene oxide to a molecule of propylene glycol. Typically these oxyalkylation steps are performed in the presence of one or more alkaline catalysts, for example potassium hydroxide and sodium hydroxide, that are then neutralized and removed from the final product.

The poloxamers of the present invention are represented by the following general structure:

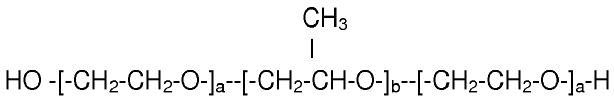

The poloxamers of the present invention are copolymers able to thicken aqueous solutions and form thermoreversible gels having reological properties varying in relation with their concentration and molecular weight.

Generally, diluted aqueous solutions of poloxamers display a newtonian flow. As an example, at concentrations above 20% in weight these solutions switch to plastic flow with substantial change of viscosity and fluidity. The influence of concentration and temperature on the viscosity of aqueous solutions of poloxamers are, for instance, reported by Lenaerts, Vincent et al. in Int. J. Pharm. 1987, 39, (1-2) 121-7. In order to better explain the present invention the content of the above cited publication is here fully incorporated.

The poloxamers utilized in the present invention have a polymeric chain of variable length. In some cases the poloxamers may be selected among Poloxamer 188 (Lutrol F 68), CAS No.9003-11-6, Poloxamer 407 (Lutrol F 127 Prill), CAS No.900-11-6, and their mixtures. By employing these specific poloxamers an optimized dose-effect ratio of the active principle, together with a suitable release time extension, can be achieved in order to favour a faster recovery of the damaged tissues.

According to one form of the invention the amount of the thickening viscous matrix contained in the pharmaceutical composition ranges between 0.2 and 40% in weight, preferably between 2 and 25%, and, even more preferably, between 5 and 22% of the overall weight of the composition.

According to some forms of the invention the topical active principle is a pharmaceutically active principle.

According to another aspect the present invention is extended to a previously described pharmaceutical composition used in odontoiatry.

### DETAILED DESCRIPTION OF THE INVENTION

In accordance with one aspect of the invention, hyaluronic acid increases the adhesion of the thermoreversible gel composition to the gingival mucosa and determines prolongation of the absorption time of the active principle. In this way the therapeutical result can be achieved at lower doses of the active principle. Hyaluronic acid, used in the present invention, is a polysaccharide with alternating 1-3 glucuronidic and 1-4 glucosaminidic bonds. The Applicants have found that the incorporation of hyaluronic acid or its derivatives into the thermoreversible gel composition determines, at human body temperature, a synergistic adhesive effect capable of maintaining the gel firmly stuck to the mucosae for periods of time longer with respect to traditional gels.

Moreover, the hyaluronic acid, present in the thermoreversible gel composition of the invention, helps to keep the gel in contact with the mucosa, extending in this way the time of release of the active principle contained into the gel and reducing the amount of active principle needed to display the searched therapeutic effect. The hyaluronic acid has also the advantage to be physiologically acceptable and fully suited with the oral mucosa, being a substance already present in some human tissues.

An additional benefit obtained from the presence of hyaluronic acid or its derivatives in the composition of the invention derives from the healing effect they display on a bleeding mucosa. In some forms of the invention, hyaluronic acid is present as a salt, for instance as the sodium or potassium salt, or as a derivative or complex with other components, for example with glucosamine.

In some forms of the invention hyaluronic acid is present into the composition of the invention in largely variable amounts ranging, for example, from 0.01 to 10% in weight. In some forms of the invention hyaluronic acid is present in amounts between 0.05 and 2%, preferably between 0.1 and 1% in weight. In some forms of the invention hyaluronic acid is present as a salt, tipically as sodium salt, in amounts sufficient to increase the viscosity of the thermoreversible gel. Hyaluronic acid is, of course, compatible with the components of the invention. Within the present invention the term ingredient or active principle refers to a substance having pharmaceutical or pharmacological effect or a biologically active substance. Biologically active substance refers to a substance that, once applied or administered topically, displays some kind of biologic action. Typical examples of biologically active substances are vitamins, natural extracts, physiologically acceptable mineral salts, such as, for example, fluorine salts.

In some forms of the invention the composition may contain a combination of active ingredients. Within the invention any combination of topically suitable active principles can be used. The present invention supplies several compositions as thermoreversible gel for the prolonged release of active principles. In one form of the invention a thermoreversible gel composition may contain up to 15% in weight of at least one topically active principle or of one of its pharmaceutically acceptable salts, between 0.2 and 40% in weight of a poloxamer, from 0.01 to 10% in weight of hyaluronic acid, the remainder being water. Typically, the amounts are expressed as per cent of the overall weight of the composition.

In other forms of the invention a thermoreversible gel composition may contain from 0.5 to 10% in weight of at least one topically active principle or of one of its pharmaceutically acceptable salts, between 2 and 25% in weight of a poloxamer, from 0.05 to 5% in weight of hyaluronic acid, the remainder being water. Typically, the amounts are expressed as per cent of the overall weight of the composition.

Typically, the water employed is deionized or anyhow suitable for pharmaceutical formulations. The amount of water present into the thermoreversible gel composition of the present invention is largely variable, for example between 10 and 95% in weight, typically between 20 and 80% in weight. In some forms of the present invention the thermoreversible gel composition may contain also a pH controlling agent in order to make it compatible with the pH of saliva.

In general, the composition of the present invention is suited for administration or topical application of any pharmaceutically or biologically active ingredient. The composition in the form of thermoreversible gel is particularly fit for topical application of at least one active ingredient selected amongst local disinfectants, fluorine based mineral salts, substances with cellular antioxidizing activity, artificial saliva, local anaesthetics, antibiotics, non steroidal (FANS) or steroidal antiinflammatory drugs and their mixtures.

In some forms of the invention the active principle is a local disinfectant, typically a chlorinated compound, such as, for example, chlorhexidine. In other forms of the invention the biologically active substance is a fluorinated compound, such as, for example, calcium fluoride, sodium fluoride or sodium mono fluorophosphate, typically in the presence of alkaline phosphatase.

In particular it has been observed that the thermoreversible gel composition enables prolonged and progressive local release of fluoride ions. In this manner the ingestion of excessive amounts of fluorine, as happens with the presently available fluorinated solutions and most importantly by children, can be avoided. In some forms of the invention the biologically active substance is a bleaching agent such as, for example, carbamide, sodium percarbonate and, in particular, their mixtures or hydrogen peroxide. In some other forms of the invention the biologically active substance includes oxalates, for instance, potassium oxalate, used in the treatment of dental hypersensitivity.

It has been observed that use of an association of carbamide and hyaluronic acid into the thermoreversible gel composition produces an efficient whitening action, reducing in the meantime the risk of damage to enamel due to massive or excessive release of whitening agent. In other forms of the invention the biologically active substance is a substance with antioxidative and/or healing activity, such as, for example, an extract of *Aloe vera* or artificial saliva.

In those forms of the invention where the active principle is a cellular antioxidative substance or a free radical scavenger, the formulation of the thermoreversible gel preparation allows to reduce the oxidative stress on the cells of treated tissues, due to external agents such as smoke, alcohol, etc. According to one form of the invention, the active principle into the thermoreversible gel composition is a local anaesthetic, such as, for example, lidocaine. Such composition can be applied to anaesthetize the gingival mucosa, for example before operations or as odontoiatric pretreatment.

According to another form of the invention, the active principle contained in the thermoreversible gel composition is a non steroidal antiinflammatory drug (FANS), such as, for example, ketorolac tromethamine, diclofenac or their mixtures. Such kind of composition can be applied in the treatment of the inflammatory forms of oral mucosae and as helper in other gingival pathologies, such as, for example, pyorrhoea.

According to another form of the invention, the active principle of the thermoreversible gel composition is an antibiotic, such as, for example, doxycycline. In this case the composition may be employed whenever there is an infection requiring topical antibiotic treatment or as helper of systemic antibiotic therapies.

According to another form of the invention, the active principle of the thermoreversible gel composition is a steroidal cortisonic drug, such as, for example, desoxymethasone.

Typically, the odontoiatric active principles are contained in the thermoreversible gel composition in pharmacologically active amounts, for instance from 0.001 to 15% in weight, conveniently from 0.1 to 5% of the overall weight of the composition.

The specific form as thermoreversible gel composition of the invention provides a prolonged release time of the active principle in the most different applications of the odontoiatric field. In some forms of the invention the composition may contain an additional muco-adhesive agent, such as a cellulose derivative, in efficacious amounts. Herein, the term muco-adhesive agent refers to a substance able to increase the adhesion of the thermosensitive gel to gingival mucosae.

In accordance with some forms of the invention, the composition may contain other components, such as stabilizers, thickeners, preservers, moisteners, dyes, commonly used in pharmaceutical preparations. In some forms of the invention, the composition may contain one or more aromatizers or natural flavours used in preparations for oral use or oral hygiene, such as, for example, mint, strawberry, anise and more others.

These further components may be present into the composition in variable amounts, according to well known tecniques employed for pharmaceutical preparations. When the topical active principle is degradable, particularly when it is contained in an aqueous colloidal system, it is possible to formulate the composition in such a way to separate the active principle from the solution containing the matrix forming the thermoreversible gel. Before or when needed, the composition may be extemporarily reconstituted.

In these cases the composition may be packaged or preserved in a device having separated containers. In particular, the degradable active principle may be placed inside a container that may be, for example, a tank or a stopper while the aqueous solution containing the thickening agent (poloxamer) may be placed in another container, typically a vial. The two containers (stopper and vial) may be separated by a septum or diaphragm that can be broken under pressure, releasing the active principle of the first container (stopper) into the aqueous solution of the second container (vial). In this way the reconstituted composition is ready to use or may be preserved for a given time in proper conditions.

The composition of the invention is used topically and may be applied either manually or using suitable odontoiatric devices, such as spatulae, syringes, appliers, etc. or may be sprayed. Once applied on the interested area, the thermoreversible gel composition of the invention may be easily eliminated typically by washing with cold water, for example at 7-10°C. In these conditions the composition switches from gel to liquid and is easily washed away.

Among the advantages derived from the use of the composition of the invention in odontoiatric or odontostomatological field are the following:
- slow release of the active principle due to the action of both hyaluronic acid and the thermoreversible gel
- keeping the active concentration of the active principle restrained only to the area needing treatment
- optimization and reduction of the amount of active principle used, due to the tenaciousness of the gel to adhere to target structures with consequent reduction of possible side effects

The present invention will be hereafter described referring to the following examples herewith intended to better illustrate the present invention without posing any limitation to it.

### EXAMPLES

### Example 1

Thermoreversible gels based on Poloxamer 407 and containing a fluorinated compound as the active principle have been prepared.

| | |
|---|---|
| Sodium fluoride | 0.15% |
| Hyaluronic acid | 0.20% |
| Poloxamer 407 | 27.00% |
| Methyl p-hydroxybenzoate | 0.18% |
| Propyl p-hydroxybenzoate | 0.02% |
| Depurated water to 100.00% | |

The thermoreversible gel has been poured in plastic moulds and applied, after jellying, on superior and inferior dental arches of children in order to provide fluorine to teeth.

The thermosensible gel at the indicated concentration has been put in contact with teeth for about 30 minutes.

Advantages: the gel, treated at temperature suited to begin solidification, is applied to dental arches where concludes the solidification and consequently does not dissolve with warmth as traditional gels. This formulation prevents the dispersion of the gel that is not swallowed by the patient. The dental adsorbtion of fluorine has been verified performing a study on extracted teeth. The presence of fluorine before and after treatment has been verified.

### CLINICAL DATA

Also the clinical study has displayed interesting results.

The second preparation is based on the presence of an alkaline phosphatase added at the moment of use to the cooled gel, in order to ease the release of fluorine from the compound containing it (sodium fluoro monophosphate).

### Example 2

Formulation of a thermoreversible gel composition containing a whitening agent as the active principle.

| | |
|---|---|
| Carbamide | 18.00% |
| Hyaluronic acid | 0.20% |
| Poloxamer F127 | 22.00% |
| Methyl p-hydroxybenzoate | 0.18% |
| Propyl p-hydroxybenzoate | 0.02% |

Depurated water to 100.00%

The used product releases free oxygen that acts in contact with the surface of teeth. The combination of two whiteners, for example carbamide and sodium perborate, synergizes the oxidizing action.

### Example 3

Formulation of a thermoreversible gel composition containing a healing and muco-adhesive agent as the active principle.

| | |
|---|---|
| *Aloe vera* | 0.10-5.00% |
| Hyaluronic acid | 0.50% |
| Poloxamer 407 | 21.56% |
| Methyl p-hydroxybenzoate | 0.18% |
| Propyl p-hydroxybenzoate | 0.02% |
| Depurated water to 100.00% | |

The *Aloe* containing thermosensible gel, with the cooperation of hyaluronic acid, remains in contact with the application area for long time, forming a protective film. The formulation presents a good stability.

### CLINICAL DATA

Clinical controls have shown adequate results.

### Example 4

Formulation of a thermoreversible gel composition containing a local anaesthetic as the active principle.

| | |
|---|---|
| Lidocaine hydrochloride | 5.00% |
| Hyaluronic acid | 0.20% |
| Poloxamer 407 | 20.75% |
| Methyl p-hydroxybenzoate | 0.18% |
| Propyl p-hydroxybenzoate | 0.02% |
| Depurated water to 100.00% | |

Lidocaine based gel formulation has the advantage to be usable also by non medical personnel. The gel contains an amount of anaesthetic used in operations that the hygienist can do (for example the "Curretage").

The thermosensible gel is sprayed on the dental mucosa where jellies at human body temperature anaesthetizing the application area with little systemic adsorbtion. The composition is stable in standard conservation conditions.

### CLINICAL DATA

The gel has been tested by applying it to the gingival sulcus of 50 patients for 10 minutes. Compared to placebo treated patients the obtained results were adequate.

### Example 5

Formulation of a thermoreversible gel composition containing an antibiotic as the active principle.

| | |
|---|---|
| Doxycycline hyclate | 3.46% (equivalent to 3.00% of base) |
| Hyaluronic acid | 0.20% |
| Poloxamer 407 e Poloxamer 188 | 21.20% |
| Methyl p-hydroxybenzoate | 0.18% |
| Propyl p-hydroxybenzoate | 0.02% |
| Depurated water to 100.00% | |

Range of concentration: 0.50 - 5.00% of antibiotic (Doxycycline base)

Range of concentration: 0.05 - 2.00% (Hyaluronic acid)

The thermosensible gel based on Polaxamer 407 contains water that causes a reduction in time of the stability of the antibiotic.

To overcome the problem a packaging has been used where the gel is conserved into a container and the antibiotic is contained, as a powder, into the stopper.

At the moment of use the antibiotic is added to the gel simply by pressing the stopper and breaking the existing diaphragma.

This device, already known for powders to be dissolved in water, cannot be used for traditional gels that are solid at low temperature and liquid after warming. The gel of the invention presents an opposite behaviour. The antibiotic is added to the cooled (4-5°C) liquid gel and dissolves uniformly.

### CLINICAL DATA

The gel has been tested on 20 patients after extraction of wisdom teeth, in parallel with patients treated orally with Rifampicin. The results have been superior and more reassuring with the Doxycycline hyclate containing gel. Stability: the whole gel is stable for about one month in the refrigerator (4-8°C). The employed packaging is suited to protect from light during utilization.

### Example 6

The composition of the gel containing a chlorinated compound is the following:

| | |
|---|---|
| Chlorhexidine gluconate | 2.00% (equivalent to 1.2% of base) |
| Hyaluronic acid | 0.20% |
| Poloxamer 407 | 20.75% |
| Methyl p-hydroxybenzoate | 0.18% |
| Propyl p-hydroxybenzoate | 0.02% |
| Peppermint aroma | 0.01% |
| Depurated water to 100.00% | |

Chlorhexidine containing thermosensible gel has the advantage to stay in contact with gums for a period of time longer than traditional gels.

It has been found that the thermosensible gel remains for a long time on the treated area, displaying an antibacterial effect superior to that obtained with traditional gels. It adheres also to wet gingival tissues with a thin film that remains even after washing with not cold water and air.

### CLINICAL DATA

The gel has been successfully tested in liquid form on 40 patients.

The composition is stable in standard conservation conditions.

### Example 7

Formulation of a thermoreversible gel composition containing a FANS as the active principle.

| | |
|---|---|
| Ketorolac tromethamine | 1.00% |
| Hyarulonic acid | 0.20% |
| Poloxamer 407 | 20.75% |
| Methyl p-hydroxybenzoate | 0.18% |
| Propyl p-hydroxybenzoate | 0.02% |
| Depurated water to 100.00% | |

Range of concentration: 0.02 - 1.50% (Ketorolac base)

Range of concentration: 0.05 - 2.00% (Hyaluronic acid)

The described gel presents the advantages shown by the gels of the previous examples.

### CLINICAL DATA

The gel has been successfully tested on 20 patients.

The composition is stable in standard conservation conditions.

### Example 8

Formulation of a thermoreversible gel composition containing an association of an antibiotic and a non steroidal antiinflammatory drug (FANS) as the active principles.

| | |
|---|---|
| Doxycycline hyclate | (equivalent to 3.00% of base) |
| Ketorolac tromethamine | 0.50% |
| Hyarulonic acid | 0.50% |
| Poloxamer 407 | 20.75% |
| Methyl p-hydroxybenzoate | 0.18% |
| Propyl p-hydroxybenzoate | 0.02% |
| Depurated water to 100.00% | |

Range of concentration: 0.10 - 6.00% (Doxycycline)

Range of concentration: 0.05 - 2.00% (Hyaluronic acid)

The preparation of the gel is extemporary.

Doxycycline is contained in the stopper of the container of the gel. At the moment of use a pressure is applied to the stopper, breaking the diaphragm that separates the powder from the previously cooled (5-8°C) gel. In this way Doxycycline mixes to the gel and dissolves in the water present.

When conserved in the refrigerator the preparation is stable for about 30 days. Preparation after reconstitution: about 30 days.

### CLINICAL DATA

The extemporary preparation has been tested on 40 patients with very good results.

### Example 9

Formulation of a thermoreversible gel composition containing a cortisonic drug as the active principle.

| | |
|---|---|
| Dexamethasone sodium phosphate | 0.20% |
| Hyarulonic acid | 0.10% |
| Poloxamer 407 | 22.00% |
| Methyl p-hydroxybenzoate | 0.18% |
| Propyl p-hydroxybenzoate | 0.02% |
| Depurated water to 100.00% | |

Range of concentration: 0.05 - 1.00% (Dexamethasone)

Range of concentration: 0.05 - 2.00% (Hyaluronic acid)

The above described gel presents the advantages of the previously described gels.

### CLINICAL DATA

The gel has been successfully tested on 20 patients.

The composition is stable in standard conservation conditions.

### Example 10

Formulation of a thermoreversible gel composition containing a non steroidal antiinflammatory drug (FANS) as the active principle.

| | |
|---|---|
| Diclofenac sodium | 1.50% |
| Hyaluronic acid | 0.50 % |
| Poloxamer 407 | 22.00% |
| Methyl p-hydroxybenzoate | 0.18% |
| Propyl p-hydroxybenzoate | 0.02% |
| Depurated water to 100.00% | |

Range of concentration: 0.05 - 3.00% (Diclofenac)

Range of concentration: 0.05 - 2.00% (Hyaluronic acid)

Stability: good if conserved as indicated.

### CLINICAL DATA

Satisfying results have been obtained from clinical controls.

### Example 11

Formulation of a thermoreversible gel composition containing artificial saliva as the active principle.

| | |
|---|---|
| Lysozyme | 0.05% |
| Lactoferrin | 0.02% |
| Lactoperoxidase | 0.02% |
| Potassium Thiocyanate | 0.01% |
| Acido laluronico | 0.05% |
| Xylitol | 1.00% |
| Potassium chloride | 0.10% |
| Methylcellulose | 0.80% |
| Aroma | 0.30% |
| Poloxamer 407 | 16.00% |
| Methyl p-hydroxybenzoate | 0.18% |
| Propyl p-hydroxybenzoate | 0.02% |
| Depurated water to 100.00% | |

### Example 12

Study of release and permeability of thermoreversible gel containing chlorhexidine digluconate.

Materials: thermosensible gel with 20% w/v Chlorhexidine digluconate.

Preparation of the mucosa - porcine oral cavity mucosa, deprived of the connective tissue, was mounted on a Franz cell, width 1.0±0.1 mm.

Permeation - this test has been performed on a modified Franz cell (Ceschel et al., **2001**). The lower compartment was filled with 4.8 mL of artificial saliva to simulate the situation inside the oral cavity, while the upper compartment was filled with 3 mL of phosphate buffer at pH 7.4 to simulate blood circulation.

The porcine oral cavity mucosa was placed in the Franz cell, keeping the external surface turned towards the lower compartment.

The gel was adherent to the external surface of the mucosa, while the solution was stirred at 600 rpm by a teflon coated magnetic bar.

The permeated amount of CHX was measured by HPLC at regular intervals of time (0.5, 1, 2, 3, 4, 5, 6, 7, 8 hours), taking samples from the upper compartment.

The experiment was run in triplicate.

HPLC (Gilson, model 305) with UV detector at 239 nm (Spectra-Physics, model Spectra 200)

Mobile phase: Acetonitrile (50%), phosphate buffer at pH 3 (50%)

Flux: 1.0 mL/min

Retention time: 5.0 min

Column: Nova-Pack C18 (150 x 3.9 mm, 4 mm, Waters)

From the permeability profile it results that chlorhexidine permeates sparingly from the tested gel, the amount not exceeding 5 µg, with a diffusion value of 10⁻⁷ cm/s. This aspect is important since the application spot is also the adsorption area. It can consequently be concluded that the active principle remains in contact with the mucosa for a prolonged period of time.

## Claims

1. A pharmaceutical composition in form of a thermoreversible gel comprising:
i) water,
ii) a topically active ingredient,
iii) a thickening viscous matrix containing at least one poloxamer,
iv) a muco-adhesive agent that sticks the gel formulation to the oral mucosa or to the dental enamel
said formulation being **characterized by** the fact that the muco-adhesive agent is hyaluronic acid or derivatives or salts thereof.

2. Composition according to claim 1, **characterized in that** the poloxamer is selected from Poloxamer 188, Poloxamer 407 and their mixtures.

3. Composition according to claim 1 or 2, **characterized in that** the hyaluronic acid or its derivatives or salts is in amount ranging from 0.01 to 10.00% in weight.

4. Composition according to claim 3, **characterized in that** the hyaluronic acid or derivatives or salts thereof is in amount ranging from 0.05 to 5.00% in weight.

5. Composition according to anyone of claims 1-4, **characterized in that** it comprises an amount of 0.05 to 10.00% by weight of at least one topically active ingredient or a pharmaceutically acceptable salt thereof, an amount of 2 to 25% by weight of a poloxamer, an amount of 0.05 to 5.00% by weight of hyaluronic acid, the remainder being water.

6. Composition according to anyone of claims 1-5, **characterized in that** said topically active ingredient is a pharmaceutically acceptable ingredient or a biologically active compound or mixtures thereof.

7. Composition according to anyone of claim 1-5, **characterized by** the fact that said topically active ingredient is selected from local disinfectants, fluorinated mineral salts, antioxidative cellular substances, artificial saliva, local anaesthetics, antibiotics, non steroidal (FANS) or steroidal anti-inflammatory drugs and their mixtures.

8. Composition according to claim 6, **characterized by** the fact that said local disinfectants are chlorinated compounds, said fluorinated mineral salts are calcium fluoride, sodium fluoride or sodium monofluorophosphate, said antioxidative cellular substances is an extract of *Aloe vera,* said local anaeshetic is lidocaine, said antibiotic is doxycycline, said FANS is ketorolac or diclofenac or mixtures thereof, said steroidal anti-inflammatory drug is dexamethasone.

9. Composition according to claim 8, **characterized by** the fact that said chlorinated compounds include chlorhexidine.

10. Composition according to anyone of claims 1-9, **characterized by** the fact that it comprises a further muco-adhesive agent, preferably selected from derivatives of cellulose.

11. Composition according to anyone of claims 1-10, **characterized by** the fact that said topically active principle is stored separately from the other components and said composition is reconstituted before use by adding the topically active ingredient to water and to the thickening viscous matrix.

12. Composition according to anyone of claims 1-11 for use in the treatment of odontoiatric or odontostomatological affections.
